# EUROPÄISCHE PATENTANMELDUNG

(11) **EP 4 656 624 A1**
(43) Veröffentlichungstag der Anmeldung: **03.12.2025**
(21) Anmeldenummer: 25178474.0
(22) Anmeldetag: 23.05.2025
(51) Int. Cl.: C07C 45/36, C07C 49/786

(54) **VERFAHREN UND ANORDNUNG ZUR HERSTELLUNG EINES OXIDIERTEN PRODUKTS**

(30) Priorität: 31.05.2024 DE 102024115267
(71) Anmelder: Forschungszentrum Jülich GmbH, 52425 Jülich (DE)
(72) Erfinder: Gundermann, Michael, 91560 Heilsbron (DE); Siegert, Fabian Hermann, 91052 Erlangen (DE); Wasserscheid, Peter, 91054 Erlangen (DE)
(74) Vertreter: Paul & Albrecht Patentanwälte PartG mbB

(57) **Zusammenfassung**

Verfahren zur Herstellung eines oxidierten Produkts, umfassend die folgenden Schritte:
- Bereitstellen eines Edukts, welches eine alkylverbrückte, insbesondere eine methylverbrückte, diaromatische oder polyaromatische Verbindung enthält oder daraus besteht;
- Verdampfen des Edukts in einer Verdampfereinrichtung (1);
- Oxidation des verdampften Edukts durch Kontakt mit einem sauerstoffhaltigen Stoff in einem Reaktor (4).

## Beschreibung

Die vorliegende Erfindung betrifft ein Verfahren zur Herstellung eines oxidierten Produkts. Ferner betrifft die Erfindung eine Anordnung zur Herstellung eines oxidierten Produkts.

Oxidierte Produkte, beispielsweise Benzophenon, werden in vielen technischen Bereichen eingesetzt. Mögliche Anwendungsgebiete sind Tinten und Beschichtungen in der Druckindustrie. Daneben ist beispielsweise Benzophenon als Inhaltsstoff in Kosmetika enthalten.

Für die Herstellung solcher oxidierten Produkte bestehen verschiedene Möglichkeiten. Benzophenon als eines von vielen Beispielen kann durch Friedel-Crafts-Alkylierung von Benzol mit Benzoylchlorid unter der Verwendung von Aluminiumchlorid hergestellt werden.

Auch wenn sich eine derartige Produktion von oxidierten Produkten grundsätzlich bewährt hat, so kann als nachteilig empfunden werden, dass relativ große Mengen an Katalysatoren und Substanzen verbraucht werden, die teilweise problematisch in der Handhabung und umweltschädlich sind. So können erhebliche Mengen an gefährlichen Abfällen anfallen, die nur schwierig zu entsorgen sind. Außerdem ist die Verfügbarkeit von einigen Rohstoffen gering.

Die Aufgabe der vorliegenden Erfindung besteht darin, ein alternatives Verfahren und eine alternative Anordnung zur Herstellung eines oxidierten Produkts zu schaffen. Bevorzugt sollen dabei die genannten Nachteile vermieden werden und insbesondere eine effiziente Herstellung eines oxidierten Produkts ermöglicht werden.

Diese Aufgabe ist bei einem Verfahren der eingangs genannten Art gelöst, welches die folgenden Schritte umfasst:
- Bereitstellen eines Edukts, welches eine carbonylverbrückte oder eine alkylverbrückte, insbesondere eine methylverbrückte, diaromatische oder polyaromatische Verbindung enthält oder daraus besteht;
- Verdampfen des Edukts in einer Verdampfereinrichtung;
- Oxidation des verdampften Edukts durch Kontakt mit einem sauerstoffhaltigen Stoff in einem Reaktor.

Diese Aufgabe ist ferner gelöst durch eine Anordnung zur Herstellung eines oxidierten Produkts, umfassend
- eine Verdampfereinrichtung zum Verdampfen eines Edukts, welches eine carbonylverbrückte oder eine alkylverbrückte, insbesondere eine methylverbrückte, diaromatische oder polyaromatische Verbindung enthält oder daraus besteht;
- einen der Verdampfereinrichtung nachgeschalteten Reaktor zur Oxidation des verdampften Edukts durch Kontakt mit einem sauerstoffhaltigen Stoff.

Der Erfindung liegt die grundsätzliche Überlegung zugrunde, ein Edukt, welches carbonylverbrückte oder alkylverbrückte diaromatische oder polyaromatische Verbindungen enthält oder daraus besteht - somit auch als ein organisches Edukt bezeichnet werden kann -, in einem ersten Schritt zu verdampfen, also in den gasförmigen Aggregatzustand zu überführen, bevor in einem zweiten Schritt das Edukt mit einem sauerstoffhaltigen Stoff in Kontakt gebracht wird und so oxidieren kann. Dadurch kann ein oxidiertes Produkt mit hoher Reinheit erzeugt werden. Als Edukt wird nachstehend das ursprüngliche Edukt bezeichnet, welches eine carbonylverbrückte oder eine alkylverbrückte, insbesondere eine methylverbrückte, diaromatische oder polyaromatische Verbindung enthält oder daraus besteht.

Bevorzugt ist das Verfahren dadurch gekennzeichnet, dass mindestens 0,1 %, insbesondere mindestens 1 %, bevorzugt mindestens 2 %, weiter bevorzugt mindestens 5 % oder 10 % der eingesetzten Eduktmenge zu Kohlenmonoxid (CO) und/oder Kohlendioxid (CO₂) oxidiert werden. Bei dem Verfahren können mehr als 5 % der Eduktmenge zu CO- und/oder CO₂ oxidiert werden, wodurch eine hohe Reinheit des oxidierten Produkts erhalten werden kann.

In weiterer Ausgestaltung kann die Verbindung Diphenylmethan, Diphenylethan, insbesondere 1,1-Diphenylethan und/oder 1,2-Diphenylethan, Diphenylpropan, insbesondere 1,2-Diphenylpropan, Fluoren, Benzyltoluol insbesondere ortho-Benzyltoluol, meta-Benzyltoluol und/oder para-Benzyltoluol, Metylbenzophenon, insbesondere 2-Metylbenzophenon, Dihydroanthrazen , insbesondere 9,10-Dihydroanthrazen, Benzylnaphtalin, insbesondere 1-Benzylnaphtalin, Dibenzyltoluol, oder Dibenzylbenzol enthalten oder daraus bestehen. Das Edukt kann auch eine Mischung aus einem oder mehreren dieser Stoffe enthalten oder daraus bestehen.

Abhängig von der Wahl der Verbindung, welche in dem Edukt enthalten ist oder aus welcher das Edukt besteht, ergibt sich dann das jeweilige oxidierte Produkt. Bei dem oxidierten Produkt kann es sich beispielsweise um Benzophenon, Fluorenon, Xanthon, Diphenylacetaldehyd, insbesondere 1,1-Diphenylacetaldehyd, Diphenylessigsäure, insbesondere 1,1-Diphenylessigsäure, Benzylphenylketon, Dibenzylketon, Methylbenzophenon, insbesondere 2-, 3- oder 4-Methylbenzophenon, Antrachinon, Benzoylnaphtalin, Dibenzoyltoluol und/oder Dibenzoylbenzol oder eine Mischung der genannten Stoffe enthalten oder daraus bestehen. Wenn Diphenylmethan als Edukt verwendet wird, kann das oxidierte Produkt beispielsweise Benzophenon, Fluorenon und/oder Xanthon sein. Wird Diphenylethan verwendet, kann das oxidierte Produkt Diphenylacetaldehyd und/oder Diphenylessigsäure sein. Mögliche Verbindungen in Edukten und daraus herstellbare oxidierte Produkte sind in nachstehender Tabelle eingeblendet.

| **Edukt** | | **Oxidiertes Produkt** |
|---|---|---|
| Diphenylmethan | → | Benzophenon |
| Diphenylmethan | | Fluorenon |
| Diphenylmethan | | Xanthon |
| 1,1-Diphenylethan | | 1,1-Diphenylacetaldehyd |
| | | 1,1-Diphenylessigsäure |
| 1,2-Diphenylethan | | Benzylphenylketon |
| 1,2-Diphenylpropan | | Dibenzylketon |
| Fluoren | | Fluorenon |
| Ortho-Benzyltoluol | | 2-Methylbenzophenon |
| Ortho-Benzyltoluol | | Anthrachinon |
| Meta-Benzyltoluol | | 3-Metyhlbenzophenon |
| Para-Benzyltoluol | | 4-Methylbenzophenon |
| 2-Methylbenzophenon | | Anthrachinon |
| 9,10-Dihydroanthracen | | Anthrachinon |
| 1-Benzylnaphtalin | | Benzoylnaphtalin |
| Dibenzyltoluol | | Dibenzoyltoluol |

| | | |
|---|---|---|
| Dibenzylbenzol | | Dibenzoylbenzol |
| Mischungen der genannten Edukte | | - |

Gemäß einer besonders bevorzugten Ausgestaltung wird als Edukt 2-Methylbenzophenon verwendet. Aus diesem kann als oxidiertes Produkt Anthrachinon produziert werden. Die beiden Bezeichnungen finden sich in der Tabelle darüber. Zum einen ist 2-Methylbenzophenon als Edukt gut verfügbar. Außerdem weist das Verfahren, wenn dieses Edukt verwendet wird, eine gute Raum-Zeit Ausbeute auf. Gleichzeitig ist die Herstellung des oxidierten Produkts Anthrachinon sauberer, insbesondere weniger giftig, als bei der klassischen Umsetzung mit Benzol und Aluminiumchlorid.

In weiterer Ausgestaltung des erfindungsgemäßen Verfahrens kann die Verdampfung des Edukts in einem Durchlaufverdampfer, insbesondere in einem Fallfilmverdampfer stattfinden. Dementsprechend kann die Verdampfereinrichtung der Anordnung als Durchlaufverdampfer, insbesondere als Fallfilmverdampfer ausgebildet sein. Bevorzugt handelt es sich bei einem Fallfilmverdampfer im Prinzip um einen Wärmetauscher. Die Verdampfung findet bevorzugt aus einem sehr dünnen, zusammenhängenden Flüssigkeitsfilm statt. Beispielsweise kann dies innerhalb eines senkrechten Rohres stattfinden. Durch die Verwendung eines Durchlaufverdampfers, insbesondere eines Fallfilmverdampfers, werden kurze Kontaktzeiten und eine gleichmäßig schonende Verdampfung sichergestellt. Dadurch werden Nachteile für die Oxidation im Reaktor minimiert. Möglich ist auch die Verwendung einer anderen Verdampfereinrichtung, welche kurze Kontaktzeiten aufweist. Beispielsweise kann es sich bei der Verdampfereinrichtung auch um einen Kesselverdampfer handeln.

Denkbar ist auch, dass die Verdampfereinrichtung und der Reaktor als Baueinheit ausgebildet sind. In diesem Fall kann beispielsweise die Verdampfung auch direkt im Reaktor stattfinden.

Bei der Verdampfung des Edukts kann ein Trägergas hinzugefügt werden. In diesem Fall wird kein reines gasförmiges Edukt, welches ausschließlich aus einer carbonyl- oder alkylverbrückten di- oder polyaromatischen Verbindung besteht, erzeugt, sondern praktisch ein Gasgemisch mit einem solchen Trägergas. Bevorzugt handelt es sich bei dem Trägergas um ein inertes Gas oder um ein Gas, welches bei den entsprechenden Temperaturen nicht mit dem Edukt reagiert.

Möglich ist auch, dass bei der Verdampfung des Edukts kein Trägergas hinzugefügt wird. In diesem Fall besteht das Gas, welches die Verdampfereinrichtung verlässt, ausschließlich aus dem verdampften (organischen) Edukt.

Die Verdampfung des Edukts findet bevorzugt oberhalb der Siedetemperatur statt, insbesondere wenn kein zusätzliches Trägergas hinzugefügt wird. Bei Hinzufügen eines Trägergases findet die Verdampfung bevorzugt oberhalb der Sättigungstemperatur statt. Siedetemperatur und Sättigungstemperatur sind bevorzugt vom verwendeten Edukt bzw. der darin enthaltenen Verbindungen abhängig. Das Edukt kann im festen oder im flüssigen Aggregatzustand bereitgestellt werden. Wenn das Edukt in einem flüssigen Aggregatzustand bereitgestellt wird, kann das flüssige Edukt in einen Trägergasstrom insbesondere über eine Venturi-Düsenanordnung eingebracht werden. Hierbei handelt es sich um eine mechanisch einfache Möglichkeit, ein Edukt, welches sich bevorzugt in einem flüssigen Aggregatzustand befindet, in einen Trägergasstrom einzubringen.

Der sauerstoffhaltige Stoff kann ein Fluid, insbesondere ein Gas sein. Mit anderen Worten kann ein sauerstoffhaltiges Fluid, insbesondere ein sauerstoffhaltiges Gas, zur Bereitstellung des für die Oxidation erforderlichen Sauerstoffs bereitgestellt bzw. eingesetzt werden. Das sauerstoffhaltige Fluid, insbesondere das sauerstoffhaltige Gas, kann feucht sein und/oder Wasser enthalten und/oder mit Wasser versetzt werden.

Bevorzugt enthält das sauerstoffhaltige Fluid, insbesondere das sauerstoffhaltige Gas, einen Anteil von mindestens 0,1 % Sauerstoff. Dabei kann es sich um den Volumenanteil und/oder den Massenanteil und/oder den Stoffmengenanteil handeln. Es hat sich gezeigt, dass bereits bei geringen Sauerstoffanteilen eine effiziente Oxidation des verdampften Edukts im Reaktor stattfinden kann. Als sauerstoffhaltiges Fluid kann auch Luft, vorzugsweise aus der Umgebung, verwendet werden. Prinzipiell kann es sich um ein Gas handeln, welches molekularen Sauerstoff enthält.

In weiterer Ausgestaltung kann das sauerstoffhaltige Fluid, insbesondere das sauerstoffhaltige Gas, aus Luft, aus einem Prozessgasstrom einer Anlage oder aus einem Tank entnommen oder daraus gewonnen werden. Dementsprechend kann die Anordnung eine Sauerstofferzeugungseinrichtung umfassen, welche insbesondere als Luftzerlegungseinrichtung ausgebildet ist oder eine solche umfasst, um ein sauerstoffhaltiges Fluid bzw. einen sauerstoffhaltigen Stoff bereitzustellen.

Die Verdampfereinrichtung und der Reaktor können durch eine Fluidleitung miteinander verbunden sein. Auf diese Weise kann das verdampfte Edukt direkt dem Reaktor zugeführt werden. Das sauerstoffhaltige Fluid kann vor dem Erreichen des Reaktors mit dem verdampften Edukt vermischt werden. In diesem Fall kann sich an die Sauerstofferzeugungseinrichtung eine Ausgangsleitung anschließen, durch welche ein sauerstoffhaltiges Fluid die Sauerstofferzeugungseinrichtung verlassen kann. Diese Ausgangsleitung kann dann in die Fluidleitung münden, zumindest kann ein Zweig der Ausgangsleitung in die Fluidleitung münden.

Alternativ oder ergänzend kann das sauerstoffhaltige Fluid direkt in den Reaktor eingebracht werden. In diesem Fall kann die Ausgangsleitung, welche sich an die Sauerstofferzeugungseinrichtung anschließt, in den Reaktor münden, zumindest kann ein Zweig der Ausgangsleitung in den Reaktor münden. Mit anderen Worten kann sich die Ausgangsleitung verzweigen, so dass ein Teil des sauerstoffhaltigen Fluids in die Fluidleitung eingeleitet wird und ein anderer Teil direkt in den Reaktor eingeleitet wird. Ein direktes Einbringen des sauerstoffhaltigen Fluids kann gleichzeitig zur Kühlung des Reaktionsgemisches genutzt werden. Denkbar sind auch Ausführungsformen, bei denen an mehreren Stellen sauerstoffhaltiges Fluid in den Reaktor eingebracht wird. Mit anderen Worten können über eine Reaktionsstrecke verteilt mehrere Eintrittspunkte in den Reaktor vorgesehen sein.

Das sauerstoffhaltige Fluid kann vorgewärmt werden, bevor es in Kontakt mit dem verdampften Edukt kommt oder dem Reaktor zugeführt wird. Bevorzugt wird das sauerstoffhaltige Fluid auf eine Temperatur oberhalb der Siedetemperatur des Edukts vorgewärmt. Die Anordnung kann eine Erwärmungseinrichtung umfassen, um das sauerstoffhaltige Fluid insbesondere auf eine Temperatur oberhalb der Siedetemperatur des Edukts vorzuwärmen.

Der sauerstoffhaltige Stoff kann auch als Feststoff ausgebildet sein. Dieser kann sich in dem Reaktor befinden und einen Katalysator enthalten oder daraus bestehen bzw. als Katalysator dienen. Dementsprechend kann der sauerstoffhaltige Stoff gleichzeitig ein Katalysator sein. Der Katalysator kann ausgebildet sein wie nachstehend beschrieben. Konkret kann der sauerstoffhaltige Stoff ein Metalloxid enthalten oder aus einem solchen bestehen.

Ein sauerstoffhaltiges Fluid kann abwechselnd mit einem nicht-sauerstoffhaltigen Fluid, bevorzugt Gas, direkt in den Reaktor eingebracht werden. Dieser Ausgestaltung liegt die Überlegung zugrunde, dass keine direkte Reaktion des sauerstoffhaltigen Fluids mit dem Edukt stattfindet, sondern dies über einen Feststoff, beispielsweise einen Katalysator, erfolgt. Das bedeutet, dass der für die Oxidation erforderliche Sauerstoff nicht direkt durch ein Fluid bereitgestellt wird, sondern durch einen Feststoff. Nachdem eine bestimmte Menge an Edukt oxidiert ist, muss dieser Feststoff praktisch reoxidiert bzw. mit Sauerstoff regeneriert werden. Dazu kann der sauerstoffhaltige Stoff in Kontakt mit einem sauerstoffhaltigen Fluid gebracht werden.

Die erfindungsgemäße Anordnung kann eine Steuereinrichtung umfassen, um die Zufuhr an Sauerstoff zu regeln. Bevorzugt ist die Steuereinrichtung ausgebildet, die Menge an sauerstoffhaltigem Fluid und/oder die Sauerstoffkonzentration des sauerstoffhaltigen Fluids einzustellen. Diese Steuereinrichtung kann als Dosierventil ausgebildet sein, welches insbesondere mechanisch und/oder elektrisch und/oder pneumatisch und/oder hydraulisch angesteuert wird. Bevorzugt ist die Steuereinrichtung in der Ausgangsleitung angeordnet.

Das sauerstoffhaltige Fluid kann auch vor dem Erreichen des Reaktors bzw. vor der Einleitung in die Fluidleitung erwärmt werden. Dementsprechend kann die erfindungsgemäße Anordnung eine Vorwärmeeinrichtung umfassen, um das sauerstoffhaltige Fluid vorzuwärmen. Bevorzugt erfolgt die Erwärmung auf eine Temperatur oberhalb der Siedetemperatur des Edukts bzw. bei Berücksichtigung des Drucks oberhalb der Sättigungstemperatur.

Wenn vor dem Erreichen des Reaktors eine Mischung mit dem sauerstoffhaltigen Fluid, insbesondere Gas, stattfindet und/oder ein Trägergas verwendet wird, kann der Anteil an gasförmigem (verdampften) Edukt in dem in den Reaktor eintretenden Gasgemisch mindestens 0,001 Vol.-%, insbesondere mindestens 0,1 Vol.-%, bevorzugt mindestens 1 Vol.-% oder 2 Vol.-% oder 5 Vol.-% oder 10 Vol.-%, und/oder höchstens 30 Vol.-%, insbesondere höchstens 20 Vol.-%, bevorzugt höchstens 15 Vol.-% oder 10 Vol.-% oder 5 Vol.-%, betragen. Der Anteil an Sauerstoff in dem in den Reaktor eintretenden Gasgemisch beträgt bevorzugt mindestens 0,1 Vol.-%, insbesondere mindestens 2 Vol.-%, und/oder höchstens 25 Vol.-%, insbesondere höchstens 20 Vol.-%. In dem Gasgemisch, welches in den Reaktor eintritt, kann Wasser enthalten sein. Ferner können zur Beeinflussung der Oxidationsreaktion weitere Stoffe hinzugefügt werden. Bevorzugt beträgt der Anteil von Wasser und weiteren hinzugefügten Stoffen höchstens 10 Vol.-%. Die Beladung in dem Gasgemisch mit Edukt kann mindestens 0,001 g/L, insbesondere mindestens 0,05 g/L, und/oder höchstens 0,85 g/L, insbesondere höchstens 0,35 g/L betragen.

In weiterer Ausgestaltung kann der Reaktor als Rohrbündelreaktor ausgebildet sein. Bevorzugt umfasst ein Rohrbündelreaktor eine Vielzahl von Rohren, die sich insbesondere parallel zueinander erstrecken können und in denen die chemische Reaktion, im vorliegenden Fall eine Oxidation, abläuft. Der die Rohre umgebende Raum ist üblicherweise mit einem Kühlmittel gefüllt oder wird von einem Kühlmittel durchflossen. Auf diese Weise eignet sich ein Rohrbündelreaktor insbesondere für stark exotherme Reaktionen, die in der Gasphase durchgeführt werden und bevorzugt eine kontinuierliche Wärmeabfuhr erfordern.

Der Reaktor kann einen Kühlmittelraum umfassen, durch welchen ein Kühlmittel strömt bzw. welcher von einem Kühlmittel durchflossen wird. Dieser Kühlmittelraum kann bei einem Rohrbündelreaktor der die Rohre umgebende Raum sein. Bevorzugt ist der Kühlmittelraum zu maximal ein Drittel seines Volumens mit einem Katalysator gefüllt. Weiter bevorzugt ist der Kühlmittelraum maximal zu einem Sechstel, insbesondere maximal einem Zehntel seines Volumens mit einem Katalysator gefüllt. Besonders bevorzugt ist eine Ausführungsform, bei welcher im Kühlmittelraum kein Katalysator vorhanden ist. Dieser Ausgestaltung liegt die Überlegung zugrunde, dass keine weitere chemische Reaktion unmittelbar im Kühlmittelraum ablaufen soll, sondern das Kühlmittel ausschließlich zur Abfuhr von Wärme verwendet werden soll.

Die bei der Oxidationsreaktion entstehende Wärme kann radial über die jeweilige Rohrwand an ein Kühlmittel übertragen werden. Möglich ist auch eine zumindest teilweise konvektive Wärmeübertragung nach dem Verlassen des Reaktors. Auch in einem solchen Fall soll die Wärme nicht für eine weitere chemische Reaktion genutzt werden, bevorzugt auch nicht über einen weiteren Kühlmittelkreislauf.

Bevorzugt wird weniger als 95%, insbesondere weniger als 90%, weiter bevorzugt weniger als 50%, 10% oder 1% der bei der Oxidationsreaktion entstehenden Wärme zum Antrieb einer weiteren chemischen Reaktion verwendet. Jedoch kann die Wärme für andere Prozesse, beispielsweise zum Verdampfen von Flüssigkeiten, beispielsweise von Edukten, verwendet werden.

Bevorzugt liegt die Reaktionstemperatur im Reaktor bei der Oxidation oberhalb der Siedetemperatur des Edukts und/oder unterhalb der Zündtemperatur des Edukts. Für den Fall, dass sich kein explosionsfähiges Gemisch einstellt, kann die Reaktionstemperatur auch oberhalb der Zündtemperatur des Edukts liegen.

Konkret kann die Reaktionstemperatur im Reaktor mindestens 200 °C, insbesondere mindestens 260 °C, und/oder höchstens 480 °C, insbesondere höchstens 360 °C betragen. Die Raumgeschwindigkeiten können mindestens 500 1/h und/oder höchstens 100.000 1/h betragen. Die Reaktion kann unter einem Druck von mindestens 0,1 bar, und/oder von höchstens 6 bar, insbesondere von höchstens 1,5 bar (jeweils absolut) ablaufen.

Bevorzugt handelt es sich bei der Oxidationsreaktion um eine exotherme Reaktion. Beispielsweise kann eine Wärme von 347 kJ/mol bei der Reaktion von Diphenylmethan zu Benzophenon und eine Wärmemenge von 6726 kJ bei der Umsetzung von Diphenylmethan zu CO₂ umgesetzt werden.

In weiterer Ausgestaltung findet die chemische Reaktion, d.h. die Oxidation unter dem Einfluss eines Katalysators statt. Dementsprechend weist der Reaktor einen Katalysator, welchen man allgemeiner als Katalysatormittel bezeichnen kann, zur Beschleunigung der Oxidation auf. Der Katalysator kann eine katalytisch aktive Komponente, insbesondere eine Metallkomponente, enthalten. Die katalytisch aktive Komponente kann Vanadium, Eisen, Molybdän, Wolfram, Titan, Zirkonium, Zinn, Cer, Mangan, Indium, Kobalt, Lithium, Kupfer, Blei, Rubidium, Antimon, Silber, Niob, Cäsium, Kalium, Magnesium, Phosphor, Silizium, Bor und/oder Titan enthalten oder daraus bestehen oder ein Gemisch dieser Elemente enthalten oder daraus bestehen.

Der Katalysator kann ein Metalloxid enthalten. Dieser Ausgestaltung liegt die Überlegung zu Grunde, für die Oxidation des Edukts zumindest teilweise den Sauerstoff aus dem Metalloxid zu verwenden. Insbesondere kann dies reversibel geschehen, das bedeutet, dass nach der Oxidation einer bestimmten Menge an Edukt ein sauerstoffhaltiges Fluid mit dem Katalysator in Kontakt gebracht wird, um das Metall wieder zu oxidieren und somit praktisch mit Sauerstoff erneut zu beladen.

Bevorzugt wird als Katalysator ein geträgertes System, welches insbesondere porös oder nichtporös ist, verwendet. Das geträgerte System kann zur Wärmeabfuhr optimiert sein.

In konkreter Ausgestaltung kann sich in den Rohren des Rohrbündelreaktors insbesondere partikelförmiges Katalysatorschüttgut befinden, welches eine katalytisch aktive Metallkomponente enthält. Bevorzugt befindet sich in den Rohren des Rohrbündelreaktors mindestens ein partikelförmiges Schüttgut. Insbesondere zur Hotspotkontrolle und/oder zur Unterstützung einer optimierten Wärmeabfuhr können mehrere unterschiedliche Katalysatoren und/oder Inertpartikel in dem Reaktor enthalten sein. Möglich ist auch, dass die Rohrinnenwände des Rohrbündelreaktors mit einer katalytisch aktiven Metallkomponente versehen sind. Der Reaktor kann Verwirbelungsmittel zum Verwirbeln von Partikeln, welche eine katalytisch aktive Metallkomponente enthalten, aufweisen. Damit existieren verschiedene Möglichkeiten, durch entsprechende Katalysatormittel, die insbesondere eine katalytisch aktive Metallkomponente enthalten, die Oxidationsreaktion erheblich zu beschleunigen.

Der Katalysator kann auch derart ausgestaltet sein, dass verschiedene katalytisch aktive Komponenten in Schichten im Reaktor aufgebracht sind, insbesondere auf den Rohrinnenseiten der einzelnen Rohre eines Rohrbündelreaktors. Beispielsweise kann es sich hier um phosphorhaltige Schichten zur Verhinderung der Weiteroxidation handeln.

Bei dem Katalysator kann es sich um ein VO_{X}-TiO₂-haltiges System handeln. Derartige Systeme sind kommerziell erhältlich. Konkret kann der Katalysator Inert- Steatite- Partikel und/oder insbesondere vorkalzinierte Katalysatorpartikel umfassen. Diese können in Schichten im Reaktor aufgeschüttet sein.

Die Katalysatorpartikel und/oder die Inert- Steatite- Partikel können als Ringpartikel ausgebildet sein und/oder in einem Verhältnis von 10 Inert-Steatite-Partikeln zu 7 Katalysatorpartikeln vorhanden sein.

Die bei der Oxidation entstehende Wärme kann über ein Wärmeträgermedium abgeführt und insbesondere für andere Verfahrensschritte verwendet werden. Dementsprechend kann der Reaktor Wärmetauschermittel aufweisen, um bei der Reaktion entstehende Wärme über ein Wärmeträgermedium abzuführen. Vor dem Hintergrund der exothermen Oxidationsreaktion, die in dem Reaktor stattfindet, eignet sich insbesondere ein Rohrbündelreaktor, da durch die Vielzahl von Rohren eine große Oberfläche existiert, über die die entstehende Wärme an ein Wärmeträgermedium abgegeben und so aus dem Reaktor abgeführt werden kann. Durch die Steuerung der Durchflussmenge an Wärmeträgermedium, welche beispielsweise über den Dosierventil stattfinden kann, kann die abgeführte Wärmemenge und damit die Temperatur im Reaktor eingestellt werden. Die Steuerung der Eintritts- und/oder der Austrittstemperatur des Wärmeträgermediums ist ebenfalls eine Möglichkeit, um die Reaktionstemperatur zu beeinflussen.

Die Anordnung kann entsprechende Sensormittel aufweisen, um die Prozessführung zu steuern. Die Sensormittel können Temperatursensoren, insbesondere im Reaktor zur Bestimmung der Reaktionstemperatur und/oder am Eingang oder am Ausgang des Reaktors, und/oder Sensoren zur Bestimmung der Zusammensetzung, insbesondere des Sauerstoffgehalts, umfassen.

Die Regelung der Reaktion kann über eine Regelung der Reaktortemperatur insbesondere in der Nähe von Hotspots erfolgen. Konkret kann abhängig von einer gemessenen Temperatur die Temperatur und/oder die Durchflussmenge von Kühlmitteln geregelt werden. Die Steuerung und/oder Regelung der Reaktion, beispielsweise der Reaktortemperatur, Hotspotausbildung, des Reaktionsfortschritts, und/oder des Produktspektrums kann über eine Vorwärmung des Edukts und/oder des sauerstoffhaltigen Gases erfolgen. Die Anordnung kann auch eine Einrichtung zur Steuerung und/oder Regelung der Oxidationsreaktion umfassen, welche insbesondere Düsen zum Eindüsen von kalter Umgebungsluft und/oder zur Kühlung von Hotspots und/oder Mittel zur Verbesserung der Reinheit und/oder zur Temperaturkontrolle aufweist. Durch niedrigere Temperaturen bei der Oxidationsreaktion können Zersetzungsprozesse minimiert werden.

Die über ein Wärmeträgermedium abgeführte Wärme kann bevorzugt den Wärmebedarf auch vorheriger oder nachgeschalteter Verfahrensschritte decken. Beispielsweise kann die abgeführte Wärme dazu verwendet werden, das Edukt zu verdampfen und/oder das sauerstoffhaltige Fluid aufzuwärmen. Insbesondere kann mindestens 1 %, bevorzugt mindestens 50 %, besonders bevorzugt mindestens 90 % des Wärmebedarfs durch die aus dem Reaktor abgeführte Wärme abgedeckt werden. Die Wärmeauskopplung am Reaktor kann bei einem Temperaturniveau von mindestens 120 °C, insbesondere von mindestens 260 °C, und/oder von höchstens 480 °C, insbesondere von höchstens 390 °C stattfinden.

Der Reaktor kann Zuführmittel aufweisen, durch welche sauerstoffhaltiges Fluid eingeleitet werden kann. Dieser Ausgestaltung liegt die Überlegung zugrunde, das sauerstoffhaltige Fluid nicht nur vor dem Erreichen des Reaktors mit dem Edukt zu vermischen bzw. beim Eintreten in den Reaktor, sondern die Möglichkeit zu schaffen, auch während der Oxidationsreaktion, die bevorzugt in den Rohren eines Rohrbündelreaktors abläuft, sauerstoffhaltiges Fluid einleiten zu können. Dadurch kann unter Umständen die Effizienz der Oxidationsreaktion weiter verbessert werden.

Nach der Oxidationsreaktion im Reaktor kann ein Gasgemisch den Reaktor verlassen. Bevorzugt sind mindestens 0,001 Mol-% CO₂ und/oder höchstens 15 Mol-% CO₂ im Gasgemisch, welches den Reaktor verlässt, enthalten.

Das den Reaktor verlassende Gasgemisch kann insbesondere unter Bildung eines Kondensats gekühlt werden. Das Kondensat kann allgemein auch als Produktgemisch bezeichnet werden. Bevorzugt enthält das Kondensat mindestens ein oxidiertes Produkt. Dementsprechend kann die erfindungsgemäße Anordnung Kondensationsmittel aufweisen, welche dem Reaktor nachgeschaltet sind, um ein Gasgemisch, welches nach der Oxidation den Reaktor verlässt, unter Bildung eines Kondensats zu kühlen. In konkreter Ausgestaltung können die Kondensationsmittel einen oder mehrere Wärmeüberträger, insbesondere Gas/Öl-Wärmeüberträger und/oder Gaskühler von insbesondere röhrenförmiger Bauart aufweisen. Hierbei handelt es sich um übliche Möglichkeiten, ein Gas bzw. ein Gas-Dampf-Gemisch zumindest teilweise in einen flüssigen Aggregatzustand umzuwandeln. Bevorzugt erfolgt eine Abkühlung auf eine Temperatur unterhalb der Siedetemperatur des oxidierten Produkts.

Prinzipiell denkbar sind auch Ausführungsformen, bei denen keine direkte Kondensation, sondern insbesondere zunächst eine Gas-Feststoff-Trennung stattfindet. Das kann bedeuten, dass sich kein flüssiges Kondensat bildet, sondern direkt ein Feststoff gebildet wird. Möglich ist auch, dass sich erst ein flüssiges Kondensat bildet, und anschließend ein Feststoff gebildet wird. Je höherschmelzender der Stoff ist, desto früher tritt der Stoff als Feststoff aus. Dieser Feststoff kann in einem folgenden Schritt wieder in einen flüssigen Aggregatzustand überführt werden. Dementsprechend können die Kondensationsmittel derart ausgestaltet sein, dass Bestandteile des den Reaktor verlassenden Gasgemischs in einen festen Aggregatzustand überführt werden, im Fall ohne die Bildung einer Flüssigkeit vor der Verfestigung praktisch resublimiert werden, und der dabei entstehende Feststoff in einem weiteren Schritt in den flüssigen Aggregatzustand unter Bildung eines Kondensats überführt werden. Konkret können die Kondensationsmittel einen Switch-Kondensator umfassen oder daraus bestehen.

Das Kondensat kann ferner nicht reagiertes Edukt und/oder weitere Nebenprodukte enthalten.

Denkbar ist auch, dass das den Reaktor verlassende Gasgemisch teilweise in einen festen Aggregatzustand überführt wird.

In einem bevorzugt weiteren Verfahrensschritt kann mindestens ein oxidiertes Produkt insbesondere aus dem Kondensat abgetrennt werden. Bevorzugt handelt es sich um genau ein oxidiertes Produkt, welches aus dem bevorzugt bereits möglichst reinen Kondensat bzw. Kondensatgemisch abgetrennt wird. Dementsprechend kann die Anordnung Separationsmittel aufweisen, um Komponenten des Gasgemischs, welches den Reaktor verlässt, zu trennen. Insbesondere können die Separationsmittel ausgebildet sein, Gase und Flüssigkeiten, insbesondere Kondensat voneinander zu trennen. Die Separationsmittel können den Kondensationsmitteln nachgeschaltet sein. Möglich ist auch, dass die Separationsmittel in einer Baueinheit mit den Kondensationsmitteln ausgebildet sind. Konkret können die Separationsmittel ausgebildet sein, Gase und Flüssigkeiten, insbesondere Kondensat von gasförmigen Bestandteilen zu trennen. Die Separationsmittel können ausgebildet sein, Flüssigkeiten, insbesondere mindestens ein oxidiertes Produkt, bevorzugt genau ein oxidiertes Produkt, aus dem Kondensat abzutrennen, und/oder Gase aus den gasförmigen Bestandteilen abzutrennen.

Vor der Abtrennung mindestens eines oxidierten Produkts kann eine thermische und/oder eine mechanische und/oder eine chemische Vorbehandlung des Gasgemischs stattfinden.

Eine gasförmige Komponente, insbesondere eine gasförmige Mischung, kann nach dem Verlassen des Reaktors und insbesondere vor, während oder nach der Kühlung in den Reaktor zurückgeführt werden. Hierbei kann es sich insbesondere um sauerstoffhaltiges Gas handeln. Dieses hat möglicherweise einen geringeren Sauerstoffanteil als ein sauerstoffhaltiges Fluid, welches direkt der Reaktion zugeführt wird. Allerdings kann der Anteil an Sauerstoff noch hinreichend hoch sein, um zur Oxidation erneut eingesetzt zu werden. Dementsprechend kann die Anordnung eine Rückführleitung aufweisen, um sauerstoffhaltiges Fluid oder Gas, insbesondere sauerstoffverarmtes Gas, nach dem Verlassen des Reaktors abzuzweigen und dem Reaktor wieder zuzuführen. Insbesondere kann die bevorzugt warme oder kalte Rückführung von sauerstoffhaltigem Gas in den Reaktor und/oder in das verdampfte Edukt zur Einstellung der Sauerstoffkonzentration genutzt werden.

In weiterer Ausgestaltung kann die Abtrennung insbesondere des oxidierten Produkts, bevorzugt aus dem Kondensat, mittels Destillation und/oder Filtration und/oder Phasentrennung stattfinden.

Bevorzugt findet die Abtrennung insbesondere des oxidierten Produkts, bevorzugt aus dem Kondensat, mittels eines Umkristallisationsprozesses, insbesondere mittels eines mehrstufigen Umkristallisationsprozesses statt. Beim Umkristallisationsprozess kann ein alkoholhaltiges und/oder ein polares oder ein unpolares Lösungsmittel verwendet werden. Bevorzugt wird ein alkanhaltiges oder ein alkenhaltiges Lösungsmittel verwendet. Dabei kann es sich insbesondere um ein Lösungsmittel aus der Gruppe Heptan bis Dekan handeln.

Das Kondensat, welches auch als kondensiertes Produktgemisch bezeichnet werden kann, kann für die Umkristallisation in einen beheizbaren Rührkessel überführt und dort mit einem polaren oder einem unpolaren Lösungsmittel, bevorzugt ein alkanhaltiges oder ein alkenhaltiges Lösungsmittel, versetzt werden. In konkreter Ausgestaltung kann es sich um ein Lösungsmittel aus der Gruppe Heptan bis Dekan handeln. Dabei ist das Löslichkeitsprodukt des zu gewinnenden Produkts zu beachten. Die eingesetzte Menge an Lösungsmitteln bei Raumtemperatur kann mindestens 5 %, insbesondere mindestens 15 %, bevorzugt mindestens 25 % unter der Lösungsmittelmenge liegen, die für eine vollständige Lösung benötigt wird. Das kondensierte Produktgemisch kann im Kessel, zusammen mit der benötigten Lösungsmittelmenge, bei Raumtemperatur gerührt werden. Durch Erwärmung der Mischung um eine Aufheiztemperaturdifferenz kann insbesondere der gesamte Feststoff oder das gesamte Kondensat in Lösung gebracht werden. Die Temperatur kann bevorzugt unter der Siedetemperatur des Lösungsmittels liegen. Bevorzugt wird der Kessel mit einem Inertgas beströmt.

Das Lösungsmittel kann bei einer Temperatur von höchstens 5° C unterhalb der Siedetemperatur mit dem Kondensat versetzt werden. Bevorzugt beträgt die Temperatur höchstens 10° C weniger als die Siedetemperatur, insbesondere höchstens 15° C weniger als die Siedetemperatur und besonders bevorzugt höchstens 20° C weniger als die Siedetemperatur des Lösungsmittels.

Anschließend kann die Lösung unter permanentem Rühren abgekühlt werden. Dabei kann das gereinigte oxidierte Produkt ausfällen. Fällt das gewonnene Produkt nicht aus, so besteht die Möglichkeit, die Kristallisation des gewünschten Produkts durch Zugabe eines Impfkeims initial zu starten. Bei dem Impfkeim kann es sich um ein bereits kristallisiertes Produkt, welches in hoher Reinheit vorliegt, handeln. Die Abkühlung kann um eine Temperatur von mindestens 30° C, insbesondere mindestens 45° C, bevorzugt mindestens 60° C stattfinden. Bevorzugt wird die Lösung um eine größere Temperaturdifferenz (Abkühltemperaturdifferenz) abgekühlt als sie zuvor erwärmt wurde (Aufheiztemperaturdifferenz). Die Abkühltemperaturdifferenz kann um mindestens das 1,1-fache, insbesondere mindestens das 1,3-fache, bevorzugt mindestens das 1,5-fache der Aufheiztemperaturdifferenz betragen. Die Abkühltemperaturdifferenz kann höchstens das 20-fache, insbesondere das 15-fache, bevorzugt höchstens das 10-fache der Aufheiztemperaturdifferenz betragen. Das oxidierte Produkt kann nach dem Umkristallisationsprozess filtriert und mit einem kalten Lösungsmittel gewaschen werden. Die erzielte Reinheit des oxidierten Produkts kann mindestens 85 %, insbesondere mindestens 95 %, bevorzugt mindestens 99,5 % betragen. Anschließend kann eine Trocknung des oxidierten Produkts stattfinden.

Schließlich kann die Abtrennung des Lösungsmittels aus der Lösung über eine Destillation stattfinden. Das zurückgewonnene Lösungsmittel kann wiederverwendet werden.

Dieser Umkristallisierungsprozess kann mehrfach wiederholt werden. Insbesondere kann er solange wiederholt werden, bis eine hinreichende Reinheit des oxidierten Produkts erreicht ist. Der Umkristallisationsprozess kann mehrfach mit einem Nebenproduktstrom erfolgen, ggf. mit Verwendung eines anderen Lösungsmittels, um das oxidierte Produkt soweit abzureichern, bis eine wirtschaftliche Abreicherung des Produkts nicht mehr sinnvoll ist.

Die Aufkonzentration der Nebenprodukte durch den Umkristallisationsprozess kann eine weitere Aufarbeitung dieser Nebenprodukte ermöglichen und zu einer weiteren wirtschaftlichen Verwertung führen. Insbesondere können die Nebenprodukte einer im Prozess integrierten thermischen Verwertungseinheit zugeführt werden.

Dazu korrespondierend kann die erfindungsgemäße Anordnung eine Umkristallisationseinrichtung umfassen, welche bevorzugt einen insbesondere beheizbaren und/oder abkühlbaren Kessel, insbesondere Rührkessel, aufweist. Die Separationsmittel können eine solche Umkristallisationseinrichtung umfassen oder daraus bestehen.

Das oxidierte Produkt kann nach dem Umkristallisationsprozess filtriert und/oder getrocknet werden. Dies kann separat geschehen oder parallel in einer entsprechenden Einrichtung stattfinden. Dadurch kann das Lösungsmittel von den Nebenprodukten getrennt werden. Der Umkristallisationsprozess kann mehrfach wiederholt werden. Insbesondere kann er so lange wiederholt werden, bis eine hinreichende Reinheit des oxidierten Produkts erreicht ist. Das abfiltrierte Produkt, welches bevorzugt als Feststoff ausgebildet ist, kann gewaschen und getrocknet werden.

Der verbleibende gasförmige Anteil nach der Trennung von Gas und Flüssigkeit kann weiterverwendet werden, beispielsweise aufgereinigt werden oder einer katalytischen Nachverbrennung zugeführt werden. Möglich ist auch, das verbleibende Gas oder ein dabei entstehendes Abgas über einen Kamin oder einen anderen Prozess aus der Anordnung auszuleiten. Dementsprechend kann die erfindungsgemäße Anordnung eine solche Nachverbrennungseinrichtung und/oder einen nachgeschalteten Kamin aufweisen. Möglich ist auch, das entsprechende Gas oder die Verbrennungsprodukte nach einer katalytischen Nachverbrennung einem anderen Prozess zuzuleiten.

Eine den Reaktor verlassende gasförmige Komponente, insbesondere eine gasförmige Mischung, kann aufgereinigt werden und/oder an eine Folgereaktion oder einen Folgeprozess, insbesondere zur Entfernung von organischen Anteilen, und/oder an die Umgebung abgegeben werden. Insbesondere kann dies nachgeschaltet zu den Kondensations- und/oder Separationsmitteln stattfinden. Dementsprechend kann die Anordnung eine Reinigungseinrichtung umfassen.

Denkbar sind auch Ausführungsformen, bei denen der Reaktor einen Hauptreaktor und einen oder mehrere Nachreaktoren umfasst. Auf diese Weise kann die Ausbeute des oxidierten Produkts gesteigert werden.

Die Anordnung, bzw. die einzelnen Komponenten der Anordnung, insbesondere der Reaktor, können explosionssicher ausgebildet sein, bzw. betrieben werden. Die Anordnung bzw. einzelne Komponenten der Anordnung, beispielsweise Rohrleitungen, bevorzugt die Fluidleitung und/oder die Ausgangsleitung und/oder die Rückführleitung, können beheizbar bzw. beheizt ausgebildet sein, um das unbeabsichtigte Kondensieren oder Verfestigen von Stoffen zu vermeiden.

### Ausführungsbeispiel:

In einer halb-technischen Laboranlage mit einem Thermoöl-gekühlten Rohrbündelreaktor, der eine Länge von 1 m und einen Durchmesser von 30 mm aufweist, wurden entsprechende Versuche durchgeführt. Das Edukt (z.B. Diphenylmethan) wurde in einem Verdampfer in die Gasphase gebracht, mit einem Gasstrom gemischt und beheizt in den Reaktor geführt. Das aus dem Reaktor austretende Gasgemisch wurde unter Bildung eines Kondensats abgekühlt, bevor anschließend Proben aus dem Kondensat entnommen wurden und die restliche Flüssigkeit über einen Zyklon vom Gas abgetrennt wurde. Ein Teil der Probe wurde in einem Lösungsmittel gelöst und die jeweiligen Bestandteile wurden gaschromatographisch analysiert. Die Konzentration von O₂, N₂, CO₂, CO und CH₄ im abgetrennten Gas wurde mittels eines Micro-GCs (Wärmeleitfähigkeitsdetektion) ermittelt.

Im Rahmen eines Beispielversuchs wurden 50 g/h Diphenylmethan, 600 nL/h Luft (273,15 K, 1,013 bar, 20 Vol.-% O₂) bei einem Absolutdruck von 1,06 bar oxidiert. Die Kühlung des Reaktors erfolgte mittels eines entsprechenden Öls. Die Reaktionstemperatur betrug ca. 329 °C. Die Ausbeute an Benzophenon betrug mehr als 75 % bei einem Umsatz von mehr als 80 %. 2,1 % des eingesetzten Diphenylmethans wurden zu CO₂ und CO total oxidiert. CH₄ konnte nicht detektiert werden. Als Katalysator wurde ein kommerziell erhältliches VO_{X}-TiO₂-haltiges System genutzt. Es wurden vierundfünfzig Schichten à 10 Inert- Steatite- Partikel und sieben vorkalzinierten Katalysatorpartikeln in dem Reaktorrohr aufgeschüttet. Die Abmaße der Ringpartikel sind 8 x 6 x 5 mm. Die Katalysatormenge betrug 198,5 g, wodurch sich eine modifizierte Verweilzeit von 0,154 (g x h)/L ergibt. Der Anteil der aktiven Komponenten im Katalysator beträgt 1/13 der Katalysatormenge. Die Raum-Zeit-Geschwindigkeit betrug 1100 1/h.

Für die weitere Ausgestaltung der Erfindung wird auf die Unteransprüche und die nachstehende Beschreibung eines Ausführungsbeispiels unter Bezugnahme auf die Zeichnung verwiesen. In der Zeichnung zeigt:

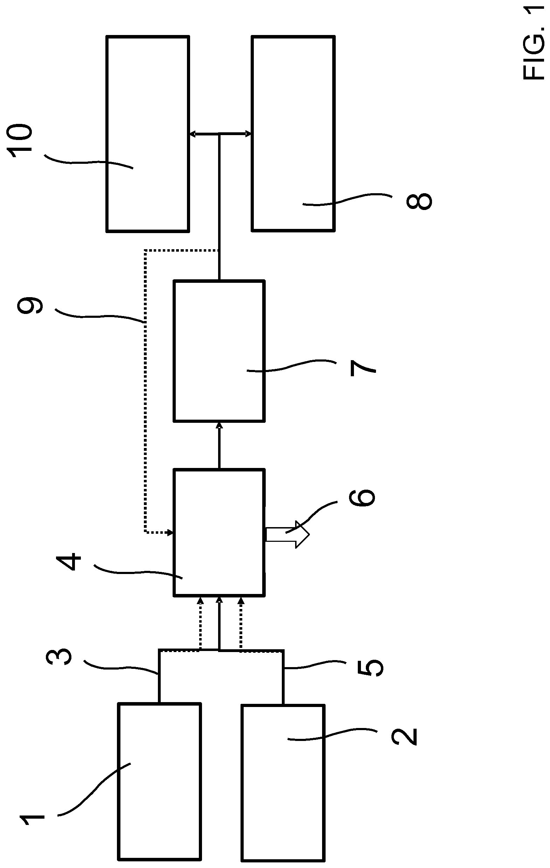

- Fig. 1: den Ablauf des erfindungsgemäßen Verfahrens in einer schematischen Darstellung.

Die Figur 1 zeigt in schematischer Darstellung den Ablauf eines erfindungsgemäßen Verfahrens. In einem ersten Schritt wird ein bereitgestelltes Edukt, welches eine alkylverbrückte, insbesondere eine methylverbrückte, diaromatische oder polyaromatische Verbindung enthält oder daraus besteht, aufgewärmt und in einer Verdampfereinrichtung 1 verdampft. Die Verdampfereinrichtung 1 kann als Fallfilmverdampfer ausgebildet sein.

Ferner wird ein nicht-sauerstoffhaltiges und/oder ein sauerstoffhaltiges Gas, dessen Sauerstoffgehalt insbesondere > 0,1 % ist, in einer entsprechenden Erwärmungseinrichtung 2 auf eine angemessene Temperatur, die bevorzugt oberhalb der Siedetemperatur des Edukts liegt, aufgewärmt. Das sauerstoffhaltige Fluid, insbesondere das sauerstoffhaltige Gas, kann feucht sein und/oder Wasser enthalten. Über eine Fluidleitung 3 ist die Verdampfereinrichtung 1 mit einem Reaktor 4 verbunden. Der Reaktor 4 ist vorliegend als Rohrbündelreaktor ausgebildet.

An die Erwärmungseinrichtung 2 schließt sich eine Ausgangsleitung 5 an, die in die Fluidleitung 3 mündet. Auf diese Weise wird das sauerstoffhaltige Gas mit dem verdampften Edukt bereits vor dem Erreichen des Reaktors 4 vermischt.

In dem Reaktor 4 wird das verdampfte Edukt durch den Kontakt mit dem sauerstoffhaltigen Gas bzw. Stoff oxidiert. Dazu wird eine katalytisch aktive Komponente, insbesondere Metallkomponente, verwendet, die beispielsweise als Schüttgut in den einzelnen Rohren des Rohrbündelreaktors angeordnet ist.

Die bei der Oxidationsreaktion entstehende Wärme, die durch den Pfeil 6 schematisch dargestellt ist, wird über ein geeignetes Wärmeträgermedium aus dem Reaktor abgeführt und kann für andere Prozessschritte, beispielsweise in der Verdampfereinrichtung 1 und/oder in der Erwärmungseinrichtung 2, verwendet werden. Die Wärme kann für weitere Prozesse verwendet werden, wodurch eine Steigerung der Effizienz der Anordnung möglich ist.

Das Gasgemisch mit dem oxidierten Produkt, welches den Reaktor 4 verlässt, wird im Anschluss durch Kondensations- und Separationsmittel 7 unter Bildung eines Kondensats abgekühlt. Insbesondere wird hier eine flüssige oder feste Phase von einer gasförmigen Phase getrennt. Die flüssige Phase bzw. die feste Phase kann weiter aufgereinigt bzw. getrennt werden, beispielsweise durch Umkristallisation in einer entsprechenden Umkristallisationseinrichtung 8. Auf diese Weise kann ein oxidiertes Produkt mit hoher Reinheit gewonnen werden.

Eine gasförmige Komponente kann über eine Rückführleitung 9 zumindest teilweise wieder dem Reaktor zugeführt werden. Auf diese Weise kann sauerstoffverarmtes Gas erneut für die Oxidationsreaktion verwendet werden. Gleichzeitig kann die Reaktionstemperatur gesenkt bzw. das in den Reaktor eintretende Edukt und/oder das sauerstoffhaltige Fluid gekühlt werden und/oder dessen Sauerstoffkonzentration eingestellt werden.

Eine verbleibende gasförmige Komponente kann in einer entsprechenden Reinigungseinrichtung 10 aufgereinigt werden und/oder einer Nachverbrennung zugeführt werden. Über einen nicht dargestellten Kamin können die verbleibenden gasförmigen Komponenten an die Umgebung abgegeben oder insbesondere als Fortluft für einen weiteren Prozess verwendet werden.

Das dargestellte Verfahren und die dargestellte Anordnung ermöglichen eine einfache und effiziente Herstellung von oxidierten Produkten aus alkylverbrückten, insbesondere methylverbrückten, diaromatischen oder polyaromatischen Verbindungen, ohne dass umweltschädliche Nebenprodukte im großen Umfang anfallen oder ein hoher Rohstoffbedarf besteht. Gleichzeitig kann die bei der Reaktion entstehende Wärme effizient für die übrigen Prozessschritte genutzt werden, so dass eine umfangreiche externe Wärmezufuhr allenfalls zum Hochfahren der Anordnung erforderlich ist.

### BEZUGSZEICHENLISTE

- 1: Verdampfereinrichtung
- 2: Erwärmungseinrichtung
- 3: Fluidleitung
- 4: Reaktor
- 5: Ausgangsleitung
- 6: Pfeil (Wärme)
- 7: Kondensations- und Separationsmittel
- 8: Umkristallisationseinrichtung
- 9: Rückführleitung
- 10: Reinigungseinrichtung

## Patentansprüche

1. Verfahren zur Herstellung eines oxidierten Produkts, umfassend die folgenden Schritte:
- Bereitstellen eines Edukts, welches eine carbonylverbrückte oder eine alkylverbrückte, insbesondere eine methylverbrückte, diaromatische oder polyaromatische Verbindung enthält oder daraus besteht;
- Verdampfen des Edukts in einer Verdampfereinrichtung (1);
- Oxidation des verdampften Edukts durch Kontakt mit einem sauerstoffhaltigen Stoff in einem Reaktor (4).

2. Verfahren nach Anspruch 1, **dadurch gekennzeichnet, dass** die Verbindung Diphenylmethan, Diphenylethan, insbesondere 1,1-Diphenylethan und/oder 1,2-Diphenylethan, Diphenylpropan, insbesondere 1,2-Diphenylpropan, Fluoren, Benzyltoluol, insbesondere ortho-Benzyltoluol und/oder meta-Benzyltoluol und/oder para-Benzyltoluol, Metylbenzophenon, insbesondere 2-Metylbenzophenon, Dihydroanthrazen, insbesondere 9,10-Dihydroanthrazen, Benzylnaphtalin, insbesondere 1-Benzylnaphtalin, Dibenzyltoluol, oder Dibenzylbenzol enthält oder daraus besteht oder eine Mischung aus einem oder mehreren dieser Stoffe enthält oder daraus besteht, und/oder dass die Verdampfung des Edukts in einem Durchlaufverdampfer, insbesondere in einem Fallfilmverdampfer stattfindet.

3. Verfahren nach einem der vorherigen Ansprüche, **dadurch gekennzeichnet, dass** bei der Verdampfung des Edukts ein Trägergas hinzugefügt wird, oder dass bei der Verdampfung des Edukts kein Trägergas hinzugefügt wird, und/oder dass das Edukt im festen oder im flüssigen Aggregatzustand bereitgestellt wird.

4. Verfahren nach einem der vorherigen Ansprüche, **dadurch gekennzeichnet, dass** der sauerstoffhaltige Stoff ein Fluid, insbesondere ein Gas ist, wobei, insbesondere, das sauerstoffhaltige Fluid, bevorzugt das sauerstoffhaltige Gas, einen Anteil von mindestens 0,1 % Sauerstoff enthält, und/oder wobei, insbesondere, das sauerstoffhaltige Fluid aus Luft, aus einem Prozessgasstrom einer Anlage oder aus einem Tank entnommen oder daraus gewonnen wird, und/oder wobei, insbesondere, das sauerstoffhaltige Fluid vor dem Erreichen des Reaktors (4) mit dem verdampften Edukt vermischt wird, und/oder wobei, insbesondere, das sauerstoffhaltige Fluid direkt in den Reaktor (4) eingebracht wird, wobei, bevorzugt, das sauerstoffhaltige Fluid abwechselnd mit einem nicht-sauerstoffhaltigen Fluid, bevorzugt Gas, direkt in den Reaktor (4) eingebracht wird.

5. Verfahren nach einem der vorherigen Ansprüche, **dadurch gekennzeichnet, dass** die Oxidation unter dem Einfluss eines Katalysators stattfindet, wobei, insbesondere, der Katalysator eine katalytisch aktive Komponente, bevorzugt eine Metallkomponente, welche Vanadium, Eisen, Molybdän, Wolfram, Titan, Zirkonium, Zinn, Cer, Mangan, Indium, Kobalt, Lithium, Kupfer, Blei, Rubidium, Antimon, Silber, Niob, Cäsium, Kalium, Magnesium, Phosphor, Silizium, Bor und/oder Titan oder ein Gemisch dieser Komponenten enthält oder daraus besteht, und/oder wobei, insbesondere, der Katalysator ein Metalloxid enthält, und/oder wobei, insbesondere, als Katalysator ein geträgertes System, welches insbesondere porös oder nichtporös ist, verwendet wird.

6. Verfahren nach einem der vorherigen Ansprüche, **dadurch gekennzeichnet, dass** bei der Oxidation entstehende Wärme über ein Wärmeträgermedium abgeführt und insbesondere für andere Verfahrensschritte verwendet wird, und/oder dass die Reaktionstemperatur bei der Oxidation oberhalb der Siedetemperatur des Edukts und/oder unterhalb der Zündtemperatur des Edukts liegt, und/oder dass eine gasförmige Komponente, insbesondere eine gasförmige Mischung, nach dem Verlassen des Reaktors (4) und vor, während oder nach der Kühlung in den Reaktor (4) zurückgeführt wird, und/oder dass eine den Reaktor (4) verlassende gasförmige Komponente, insbesondere eine gasförmige Mischung, aufgereinigt, und/oder an eine Folgereaktion oder einen Folgeprozess, insbesondere zur Entfernung von organischen Anteilen, und/oder an die Umgebung abgegeben wird.

7. Verfahren nach einem der vorherigen Ansprüche, **dadurch gekennzeichnet, dass** ein Gasgemisch nach der Oxidation den Reaktor (4) verlässt, welches unter Bildung eines Kondensats gekühlt wird, wobei das Kondensat mindestens ein oxidiertes Produkt enthält, wobei, insbesondere, das Kondensat ferner nicht reagiertes Edukt und/oder weitere Nebenprodukte enthält.

8. Verfahren nach Anspruch 7, **dadurch gekennzeichnet, dass** in einem weiteren Verfahrensschritt mindestens ein oxidiertes Produkt aus dem Kondensat abgetrennt wird, wobei, insbesondere, vor der Abtrennung mindestens eines oxidierten Produkts eine thermische und/oder eine mechanische und/oder eine chemische Vorbehandlung des Gasgemischs stattfindet, und/oder wobei, insbesondere, die Abtrennung mittels Destillation und/oder Filtration und/oder Phasentrennung stattfindet, und/oder wobei, insbesondere, das oxidierte Produkt insbesondere nach einen Umkristallisationsprozess filtriert und/oder das abfiltrierte Produkt, welches bevorzugt als Feststoff ausgebildet ist, gewaschen und getrocknet wird.

9. Verfahren nach Anspruch 8, **dadurch gekennzeichnet, dass** die Abtrennung mittels eines Umkristallisationsprozesses, insbesondere mittels eines mehrstufigen Umkristallisationsprozesses stattfindet, wobei, insbesondere, beim Umkristallisationsprozess ein alkoholhaltiges und/oder polares und/oder unpolares Lösungsmittel und/oder ein alkanhaltiges oder ein alkenhaltiges Lösungsmittel, bevorzugt Heptan bis Dekan, verwendet wird.

10. Anordnung zur Herstellung eines oxidierten Produkts, umfassend
- eine Verdampfereinrichtung (1) zum Verdampfen eines Edukts, welches eine alkylverbrückte, insbesondere eine methylverbrückte, diaromatische oder polyaromatische Verbindung enthält oder daraus besteht;
- einen der Verdampfereinrichtung (1) nachgeschalteten Reaktor (4) zur Oxidation des verdampften Edukts durch Kontakt mit einem sauerstoffhaltigen Stoff.

11. Anordnung nach Anspruch 10, **dadurch gekennzeichnet, dass** die Anordnung eine Sauerstofferzeugungseinrichtung umfasst, welche insbesondere als Luftzerlegungseinrichtung ausgebildet ist oder eine solche umfasst, um ein sauerstoffhaltiges Fluid, insbesondere ein sauerstoffhaltiges Gas, bereitzustellen.

12. Anordnung nach Anspruch 11, **dadurch gekennzeichnet, dass** sich an die Sauerstofferzeugungseinrichtung eine Ausgangsleitung (5) anschließt, durch welche ein sauerstoffhaltiges Fluid die Sauerstofferzeugungseinrichtung verlassen kann, wobei die Ausgangsleitung (5) in eine Fluidleitung (3), durch welche die Verdampfereinrichtung (1) und der Reaktor (4) miteinander verbunden sind, und/oder in den Reaktor (4) mündet.

13. Anordnung nach einem der Ansprüche 10 bis 12, **dadurch gekennzeichnet, dass** diese eine Steuereinrichtung umfasst, um die Zufuhr an Sauerstoff zu regeln, wobei die Steuereinrichtung ausgebildet ist, die Menge an sauerstoffhaltigem Fluid und/oder die Sauerstoffkonzentration des sauerstoffhaltigen Fluids einzustellen, wobei die Steuereinrichtung bevorzugt als Dosierventil ausgebildet ist, welches insbesondere in der Ausgangsleitung (5) angeordnet ist, und/oder dass der Reaktor (4) als Rohrbündelreaktor ausgebildet ist, und/oder dass der Reaktor (4) Zuführmittel aufweist, durch welche sauerstoffhaltiges Fluid eingeleitet werden kann, und/oder dass der Reaktor (4) Wärmetauschermittel aufweist, um bei der Oxidation entstehende Wärme über ein Wärmeträgermedium abzuführen, und/oder dass die Anordnung Kondensationsmittel (7) aufweist, welche dem Reaktor (4) nachgeschaltet sind, um ein Gasgemisch, welches nach der Oxidation den Reaktor (4) verlässt, unter Bildung eines Kondensats zu kühlen, wobei, insbesondere, die Kondensationsmittel (7) einen oder mehrere Gas/Öl-Wärmeüberträger und Gaskühler von insbesondere röhrenförmiger Bauart aufweisen.

14. Anordnung nach einem der Ansprüche 10 bis 13, **dadurch gekennzeichnet, dass** der Reaktor (4) einen Katalysator zur Beschleunigung der Oxidation enthält, wobei, insbesondere, sich in den Rohren des Rohrbündelreaktors bevorzugt partikelförmiges Katalysatorschüttgut befindet, welches eine katalytisch aktive Komponente, bevorzugt eine Metallkomponente, enthält, und/oder wobei, insbesondere, die Rohrinnenwände des Rohrbündelreaktors mit einer katalytisch aktiven Komponente, bevorzugt eine Metallkomponente, versehen sind, und/oder wobei, insbesondere, der Reaktor (4) Verwirbelungsmittel zum Verwirbeln von Partikeln, welche eine katalytisch aktive Komponente, bevorzugt eine Metallkomponente, enthalten, aufweist, wobei, bevorzugt, die katalytisch aktive Komponente, insbesondere Metallkomponente, Vanadium, Eisen, Molybdän, Wolfram, Titan, Zirkonium, Zinn, Cer, Mangan, Indium, Kobalt, Lithium, Kupfer, Blei, Rubidium, Antimon, Silber, Niob, Cäsium, Kalium, Magnesium, Phosphor, Silizium, Bor und/oder Titan oder ein Gemisch dieser Elemente enthält oder daraus besteht, und/oder wobei, bevorzugt, die katalytisch aktive Metallkomponente ein Metalloxid enthält.

15. Anordnung nach einem der Ansprüche 10 bis 14, **dadurch gekennzeichnet, dass** diese Separationsmittel (7) aufweist, wobei die Separationsmittel bevorzugt den Kondensationsmitteln nachgeschaltet oder in einer Baueinheit mit diesen ausgebildet sind, wobei, insbesondere, die Separationsmittel (7) ausgebildet sind, Gase und Flüssigkeiten, bevorzugt Kondensat von gasförmigen Bestandteilen, zu trennen, und/oder Flüssigkeiten aus dem Kondensat abzutrennen, und/oder Gase aus den gasförmigen Bestandteilen abzutrennen, und/oder wobei, insbesondere, die Anordnung eine Rückführleitung (9) aufweist, um sauerstoffverarmtes Gas nach dem Verlassen des Reaktors (4) abzuzweigen und dem Reaktor wieder zuzuführen.
